# EUROPEAN PATENT SPECIFICATION

(11) **EP 3 636 305 B1**
(45) Date of publication and mention of the grant of the patent: **04.05.2022**
(21) Application number: 19799860.2
(22) Date of filing: 11.01.2019
(51) Int. Cl.: A61M 11/00, A61M 15/00

(54) **MEDICAL NEBULIZER**
MEDIZINISCHER VERNEBLER
NÉBULISEUR MÉDICAL

(30) Priority: 11.05.2018 CN 201810447186
(43) Date of publication of application: 15.04.2020
(73) Proprietor: Taian Dalu Medical Instrument Co., Ltd., Taian, Shandong 271000 (CN)
(72) Inventor: CUI, Xianwei, Taian, Shandong 271000 (CN); WANG, Yuqin, Taian, Shandong 271000 (CN); LU, Baoyu, Taian, Shandong 271000 (CN); ZHOU, Senlin, Taian, Shandong 271000 (CN); LI, Huaidong, Taian, Shandong 271000 (CN)
(74) Representative: Gong, Jinping
(86) International application number: PCT/CN2019/071274
(87) International publication number: WO 2019/214281

(56) References cited:
- EP-A1- 1 743 671
- EP-A1- 2 371 409
- EP-A1- 2 868 339
- EP-A1- 2 959 978
- EP-A1- 2 959 978
- WO-A1-2012/046220
- CN-A- 107 626 021
- CN-A- 107 626 021
- CN-A- 108 379 702
- CN-U- 201 524 332
- CN-U- 202 877 026
- CN-U- 205 146 559
- CN-U- 205 146 559
- CN-U- 205 156 230
- US-A1- 2006 207 591

## Description

### TECHNICAL FIELD OF THE INVENTION

The present invention relates to a medical atomizer, in particular to a medical atomizer for atomization inhalation therapy.

### BACKGROUND OF THE INVENTION

Atomization inhalation therapy is to disperse drugs into tiny droplets or particles by an atomization device. These tiny droplets or particles are suspended in the air and sent into the respiratory tract and lungs, so as to clean and humidify the airway, realize local treatment and systemic treatment. Atomization inhalation therapy has been gradually applied in clinical treatment, which is usually implemented by a medical atomizer.

CN206007737U discloses a medical breathing machine atomizer device, which includes atomization generating device, cavity for storage medicine and three-way pipe. In this device, it does not mention detail structure of the atomization generating device. Further, its sealing ring is only used to seal the pipeline. CN103977489A discloses an atomizer for an integrated pipeline, which comprises a joint, a liquid drug storage device and a miniature actuating chip, wherein the joint is of a three-way structure with hollow inside, main body of the joint is provided with three end openings, the first end opening and the second end opening are standard connectors, the third end opening is used as a connecting end opening; the liquid drug storage device is detachably connected with the third end opening of the joint in a sealed way, and the miniature actuating chip is arranged at a liquid medicine outlet part of the liquid drug storage device and atomizes the liquid medicine entering the joint. Due to performance characteristics of the miniature actuating chip, it needs to be fixed very stable. To achieve this, a lining is arranged between the miniature actuating chip and the third end of the pipeline joint. The top end of the lining matches with the miniature actuating chip, and the bottom end is sealed with the third end of the pipeline joint through a sealing ring. Although the above-mentioned structure can protect the miniature actuating chip and prevent leakage of the atomized gas, the production and assembly efficiency is low, and the sealing performance still needs to be further improved.

CN107626021A discloses an aerosol storage type aerosolization doser, which comprises a horizontal inhalation barrel communicated with an electronic type atomizer nozzle. The other end of the horizontal inhalation barrel is perpendicularly communicated with the upper part of a vertical barrel; a one-way valve part I is arranged under the vertical barrel; an oblique inhalation barrel is communicated above the vertical barrel; a front section of the oblique inhalation barrel is communicated with an inhalation nozzle part; a one-way valve part II is arranged at the rear part of the oblique inhalation barrel. The aerosolization doser only relates to a vibration component comprising a vibrating atomizing sheet, but does not involve the protection and installation of the vibration component. In addition, the horizontal inhalation barrel may increase the contact between the atomized drug and the barrel wall, resulting in the formation of droplets, so that the atomized drug cannot be fully utilized.

In summary, an atomizer with higher assembly efficiency and higher liquid medicine utilization rate is urgently needed.

EP2371409A1 describes an apparatus for use in insufflation of a body cavity. CN205146559U relates to an atomization component and an atomizer provided with the component. EP2868339A1 describes a system for delivery of aerosol therapy.

US2006207591 discloses an aerosol generating means for inhalation therapy devices, comprising an oscillatable assembly having a membrane, to which a liquid can be supplied for generation of an aerosol, and an oscillation generating means, by means of which the membrane can be caused to oscillate for generation of an aerosol, and comprising an encapsulating means for accommodating and mounting the oscillatable assembly.

### SUMMARY OF THE INVENTION

An objective of the present invention is to provide a medical atomizer, for atomization inhalation therapy as specified in any of claims 1 to 8, which has a higher production and assembly efficiency and a higher utilization rate of liquid medicine. A further objective of the present invention is to provide a medical atomizer, which has further improved sealing property and is easy to use. The present invention utilizes the following technical solution to achieve the above purposes.

A medical atomizer comprises a drug storage device, an atomized drug collecting tank and an atomization generating assembly, an accommodation chamber is formed between the lower part of the drug storage device and the atomized drug collecting tank, the accommodation chamber is used for accommodating the atomization generating assembly;
wherein the atomization generating assembly comprises a sealing sleeve and an atomizing sheet; the sealing sleeve has a sidewall, a first panel and a second panel, the first panel and the second panel are arranged oppositely, the sidewall is arranged to connect the first panel and the second panel into an integral structure, the sidewall, the first panel and the second panel are jointly enclosed, forming a atomizing sheet chamber which is used to accommodate the atomizing sheet; the sidewall is provided with an opening for installing the atomizing sheet into the atomizing sheet chamber; the atomizing sheet is located between the first panel and the second panel, and it does not exceed the area defined by the sidewall; the middle part of the first panel is provided with a first opening, the middle part of the second panel is provided with a second opening; the first opening and the second opening are used cooperatively so as to make at least a part of the atomizing sheet expose outside the atomizing sheet chamber;
wherein the shape of the atomized drug collecting tank is spindle.

In accordance with the medical atomizer of the present invention, preferably, the lower part of the drug storage device is provided with a first socket structure, the atomized drug collecting tank comprises an upper atomized drug collecting section and a lower atomized drug collecting section, the upper part of the upper atomized drug collecting section is provided with a second socket structure, the first socket structure forms a socket connection with the second socket structure; both the first socket structure and the second socket structure have a height of 1-20 mm.

In accordance with the medical atomizer of the present invention, preferably, the medical atomizer also comprises a three-way pipe, a one-way valve and a mouthpiece; the three-way pipe comprises a first end, a second end and a third end, the first end is connected with the lower part of the lower atomized drug collecting section, the second end is open to the atmosphere, and the third end is communicated with the mouthpiece; the one-way valve is located in the three-way pipe, which is arranged to be able to lead the atomized drug in the atomized drug collecting tank out to the mouthpiece and prevent air from entering the atomized drug collecting tank.

In accordance with the medical atomizer of the present invention, preferably, the upper part of the drug storage device comprises an atomizing cup body, an atomizing cup cover, a sealing member, a clasp and a plugging component; the atomizing cup cover is arranged above the atomizing cup body and is connected with the atomizing cup body; the sealing member is arranged on the lower surface of the atomizing cup cover for sealing the atomizing cup body; the clasp is arranged on the side of the atomizing cup cover or the atomizing cup body; the plugging component is arranged on one side of the atomizing cup body, and is connected with the accommodation chamber.

In accordance with the medical atomizer of the present invention, preferably, the medical atomizer also comprises a power supply, a wire and a plug; the power supply is connected with the plug through the wire; and the plugging component is arranged to be able to accommodate the plug, and be able to electrically connect the atomizing sheet with the plug.

In accordance with the medical atomizer of the present invention, preferably, both the first panel and the second panel are in annular shape, the sidewall is a circumferential sidewall; and the diameter of the first opening is different from that of the second opening.

In accordance with the medical atomizer of the present invention, preferably, the sealing sleeve has a diameter of 10-50 mm and a thickness of 1-10 mm, and the angle of the opening on the sidewall is 30-150 degrees.

In accordance with the medical atomizer of the present invention, a first sealing ring is arranged on the inner side of the first opening, and a second sealing ring is arranged on the inner side of the second opening; the first sealing ring and the second sealing ring are matched for fixing the atomizing sheet in the atomizing sheet chamber.

In accordance with the medical atomizer of the present invention, preferably, both the first sealing ring and the second sealing ring are O-sealing rings with diameters of 2-10 mm and line diameters of 0.5-4 mm.

In accordance with the medical atomizer of the present invention, the sidewall, the first panel, the second panel, the first sealing ring and the second sealing ring are formed into integral structure; and the first panel and/or the second panel are provided with a notch which is located at the opening on the sidewall.

In the present invention, a specific sealing sleeve is used together with an atomizing sheet. The sealing sleeve and the atomizing sheet can easily form an atomizing generating component, thereby the production and assembly efficiency is improved. Further, in the present invention, an atomized drug collecting tank with a special shape is utilized, so that the atomized medicine liquid is difficult to form droplets, thereby the utilization rate of medicine is improved. In the present invention, by arranging a sealing ring on the first panel or the second panel, the atomizing sheet can be tightly combined with the sealing sleeve, and the sealing sleeve can be tightly combined with the drug storage device and the atomized drug collecting tank, thereby the sealing performance of the medical atomizer is improved. In the present invention, the atomization part and the power supply are designed separately, so that it is convenient to be used.

### DESCRIPTION OF DRAWINGS

Fig. 1 is a schematic diagram of a medical atomizer of the present invention.
Fig. 2 is a schematic diagram of a sealing sleeve of an atomization generating assembly of the present invention.
Fig. 3 is a schematic diagram of an atomizing sheet of an atomization generating assembly of the present invention.

The reference numbers are as follows:
100-atomization generating assembly, 101-sealing sleeve, 102-atomizing sheet, 200-drug storage device, 201-atomization cup body, 202-atomization cup cover, 203-sealing member, 204-clasp, 205-plugging component, 300-atomized drug collecting tank, 301-upper atomized drug collecting section, 302-lower atomized drug collecting section, 400-three-way pipe, 600-mouthpiece, 700-power supply, 800-wire, 900-plug.
1-microporous membrane, 2-conductive film, 3-silicon rubber sheath, 4-conductive spring.
11-sidewall, 12-first panel, 13-second panel, 14-first sealing ring, 15-second sealing ring, 17-opening, 18-notch, 19-first opening, 20-second opening.

### DETAIL DESCRIPTION OF THE INVENTION

The present invention will be further explained in combination with specific embodiments, but the protection scope of the present invention is not limited thereto.

The medical atomizer in the present invention can be applied in atomization inhalation therapy or other auxiliary treatment. It is totally different from the electronic atomization device of smoke, from which there is no enlightenment the inventors can learn.

The medical atomizer in the present invention comprises a drug storage device, an atomized drug collecting tank, an atomization generating assembly, and auxiliary components, such as a three-way pipe, an one-way valve, a mouthpiece, a power supply, a wire, a plug, etc. The present invention is described in details as following.

The medical atomizer in the present invention comprises a drug storage device, an atomized drug collecting tank, and an atomization generating assembly. The lower part of the drug storage device and the atomized drug collecting tank forms an accommodation chamber for accommodating the atomization generating assembly; wherein the atomization generating assembly comprises a sealing sleeve and an atomizing sheet; the sealing sleeve has a sidewall, a first panel and a second panel, the first panel and the second panel are arranged oppositely, the sidewall is arranged to connect the first panel and the second panel into an integral structure, the sidewall, the first panel and the second panel are jointly enclosed, forming an atomizing sheet chamber which is used to accommodate the atomizing sheet; the sidewall is provided with an opening for installing the atomizing sheet into the atomizing sheet chamber; the atomizing sheet is located between the first panel and the second panel, and it does not exceed the area defined by the sidewall; the middle part of the first panel is provided with a first opening, the middle part of the second panel is provided with a second opening; the first opening and the second opening are used cooperatively so as to make at least a part of the atomizing sheet expose outside the atomizing sheet chamber;

### <Drug Storage Device and Atomized Drug Collecting Tank>

The drug storage device in the present invention comprises an upper part and a lower part, both of which are interconnected. An accommodation chamber is formed between the lower part of the drug storage device and the atomized drug collecting tank for accommodating the atomization generating assembly. The upper part of the drug storage device comprises an atomizing cup body, an atomizing cup cover, a sealing member, a clasp and a plugging component. The atomization cup body and the atomization cup cover cooperate with each other to form a drug storage chamber. The atomizing cup cover may be arranged above the atomizing cup body and is connected with the atomizing cup body. There is no special restriction on the shape of the atomization cup body and the atomization cup cover. The cross-section of the atomizing cup body may be circular, rectangular, nearly rectangular, etc. Similarly, the cross-section of the atomizing cup cover may be circular, rectangular, nearly rectangular, etc.

In the present invention, the sealing member is arranged on the lower surface of the atomizing cup cover for sealing the atomizing cup body. Examples of sealing members include, but are not limited to, silicon rubber sealing ring. The sealing member is arranged between the atomizing cup body and the atomizing cup cover, and are simultaneously closely connected with the atomizing cup body and the atomizing cup cover, thus these two parts are fully sealed.

In the present invention, the clasp is arranged on the side of the atomizing cup cover or the atomizing cup body. In an example, a clasp is arranged on the side of the atomizing cup cover, and a concave edge is arranged on the upper edge of the atomizing cup body, so the clasp is matched with the concave edge, so that the atomizing cup cover and the atomizing cup body are fixed together. In another example, a clasp is arranged on the side of the atomizing cup cover, and a protrusion is arranged on the side of the atomizing cup body, so the clasp is matched with the protrusion, so that the atomizing cup cover and the atomizing cup body are fixed together. Other clasping structures known in the field can also be used, which will not be described in details herein.

In the present invention, the plugging component is arranged on one side of the atomizing cup body, and is connected with the accommodation chamber. Preferably, the plugging component and the atomizing cup body are formed into integral structure. The plugging component is used to receive the plug, thereby supplying electrical power to the atomization generating component in the accommodation chamber. The shape of the plugging component matches the shape of the plug. There are no special restrictions on the shape of plugging component, for example, it is a hollow rectangle or nearly rectangle.

In the present invention, socket connection can be applied between the drug storage device and the atomized drug collecting tank. An accommodation chamber is formed between the drug storage device and the atomized drug collecting tank for accommodating the atomization generating assembly. A medical atomizer can be easily assembled by putting the atomization generating assembly into the accommodation chamber which is formed between the drug storage device and the atomized drug collecting tank, thereby the production and assembly efficiency is improved.

The traditional atomized drug collecting tank is cylindrical, and the atomized drug (the atomized liquid medicine) contacts the wall of the cylinder, resulting in that part of atomized drug forms droplets, which cannot be effectively inhaled. In the present invention, the atomized drug collecting tank is in the shape of spindle. The spindle described herein does not mean to strictly limit its shape, the shape is acceptable as long as the structure is in a similar shape. The spindle is characterized by large middle part and two small ends. The inventors are surprised to find that such a structure can effectively reduce the contacts between the atomized drug and the atomized drug collecting tank, thereby avoiding the formation of droplets of the atomized drug which are unable to be effectively inhaled, so that the utilization rate of the drug is effectively improved.

In the present invention, the atomized drug collecting tank comprises an upper atomized drug collecting section and a lower atomized drug collecting section which are connected with each other through threads. According to an embodiment of the present invention, the upper atomized drug collecting section has internal threads, and the lower atomized drug collecting section has external threads which match with the internal threads. According to another embodiment of the present invention, the upper atomized drug collecting section has external threads, and the lower atomized drug collecting section has internal threads which match with the external threads. The combination of internal threads and external threads makes the upper atomized drug collecting section and lower atomized drug collecting section integrated and sealed. This kind of assembly is very convenient and efficient.

In some embodiment, the lower part of the drug storage device is provided with a first socket structure; the atomized drug collecting tank is provided with a second socket structure, and the first socket structure forms a socket connection with the second socket structure. According to an embodiment of the present invention, the lower part of the drug storage device is provided with a first socket structure, the upper part of the upper atomized drug collecting section is provided with a second socket structure, and the first socket structure forms a socket connection with the second socket structure. The first socket structure and the second socket structure cooperate with each other to form an accommodation chamber for accommodating the atomization generating assembly. Both the first socket structure and the second socket structure have a height of 1-20 mm, preferably 2-15 mm.

### <Atomization Generating Assembly>

The atomization generating assembly in the invention comprises a sealing sleeve and an atomizing sheet. The atomizing sheet may be accommodated in the sealing sleeve to form a complete atomizing generating assembly. In the present invention, a combination of a specific sealing sleeve and an atomizing sheet is utilized, which can easily form an atomizing generating assembly, so that the production and assembly efficiency is improved.

The atomization generating assembly in the present invention comprises a sealing sleeve and an atomizing sheet; the sealing sleeve has a sidewall, a first panel and a second panel, the first panel and the second panel are arranged oppositely, the sidewall is arranged to connect the first panel and the second panel into an integral structure, the sidewall, the first panel and the second panel are jointly enclosed, forming an atomizing sheet chamber which is used to accommodate the atomizing sheet; the sidewall is provided with an opening for the atomizing sheet to be installed into the atomizing sheet chamber; the atomizing sheet is located between the first panel and the second panel, and it does not exceed the area defined by the sidewall; the middle part of the first panel is provided with a first opening, the middle part of the second panel is provided with a second opening; the first opening and the second opening are used cooperatively so as to make at least a part of the atomizing sheet exposed outside the atomizing sheet chamber.

There is no special limitation on the atomizing sheet of the present invention. Those known in the field can be used as an atomizing sheet, such as those with microporous membranes made of polymer materials or brake chips made of stainless steel. The atomizing sheet is described following with an example of those with microporous membranes made of polymer materials. The atomizing sheet comprises microporous film, conductive sheet and silicon rubber sheath. Optionally, it may also comprise two conductive springs. The microporous film is arranged in the center of the conductive sheet and electrically connected with the conductive sheet. The conductive sheet is arranged in the silicon rubber sheath. There are many micropores on the microporous membrane, which can fluctuate with electricity, and squeeze the liquid that contacts, and then atomize the liquid to form mist. Two conductive springs are weld on the conductive sheet. The conductive spring contacts with the plug inserted into the plugging component to realize the electrical connection for the atomizing sheet. The conductive sheet supplies power to the microporous membrane from its surroundings, which ensures the stable working current for the microporous membrane and does not affect the atomization action of the microporous membrane. Microporous membranes can be made of conventional polymer materials.

The sealing sleeve in the present invention has sidewall, first panel and second panel. The first panel and the second panel are arranged oppositely. The so-called "first" and "second" do not intend to limit the number of panels, but only to mean that they are different components. Obviously, the positions of the first panel and the second panel are opposite. If the first panel is the upper panel, the second panel is the lower panel; if the second panel is the upper panel, the first panel is the lower panel. The sidewall is arranged to connect the first panel and the second panel into an integral structure. The so-called integral structure means that the sidewall, the first panel and the second panel form a solid whole. The sidewall, the first panel and the second panel are integral structure. The material of the sidewall, the first panel and the second panel can be the same or different. Preferably, the material of the sidewall, the first panel and the second panel are the same. These materials include, but are not limited to, polymer materials such as silicon rubber. The material can be elastic so as to be easy to install.

According to an embodiment of the present invention, a plurality of through holes are arranged on the first panel or the second panel. According to another embodiment of the present invention, a plurality of through holes are arranged on both the first panel and the second panel. There is no special restriction on the number and shape of through holes. In this way, some materials can be saved without affecting the installation of atomizing sheet.

In the present invention, the sidewall, the first panel and the second panel are jointly enclosed to form an atomizing sheet chamber. This atomizing sheet chamber is used to accommodate the atomizing sheet. Preferably, the shape of the atomizing sheet chamber matches the shape of the atomizing sheet. The size of the atomizing sheet chamber may be larger than that of the atomizing sheet; preferably, the size of the atomizing sheet chamber may be slightly larger than that of the atomizing sheet. For instance, in the case of that the atomizing sheet chamber is circular and the atomizing sheet is also circular, the diameter of the atomizing sheet chamber (D1) may be larger that of the atomizing sheet (D2); preferably, the diameter of the atomizing sheet chamber (D1) may be slightly larger that of the atomizing sheet (D2). For example, D1-D2 is 0.1-10 mm, preferably 0.1-5 mm.

In some embodiment, the first panel and the second panel are annular structure, and the sidewall is circumferential. In this way, a cylindrical atomizing sheet chamber can be formed. The annular structure has the common meaning in this field, i.e. a circular hole is arranged in the middle. According to an embodiment of the present invention, the first panel and the second panel are annular structures, the sidewall is circumferential sidewall; and the diameter of the first opening is different from that of the second opening.

In the present invention, the diameter of the sealing sleeve can be 10-50 mm, preferably 15-25 mm. The thickness of the sealing sleeve can be 1-10 mm, preferably 2-5 mm. In some cases, the diameter of the circumferential sidewall is equal to the diameter of the sealing sleeve, and the height of the circumferential sidewall is equal to the thickness of the sealing sleeve. The inventors of the present invention discover that such a size range is very beneficial to the vibration of the microporous film of the atomizing sheet, so that the atomizing effect is improved. In the present invention, an opening is arranged on the sidewall for the atomizing sheet to be installed in the atomizing sheet chamber. There is no special restriction on the shape of the opening. For example, a lack and an omission of part on the sidewall can form an opening. The angle of the opening can be 30-150°, preferably 90-120°, so that it is easy to install the atomizing sheet in the atomizing sheet chamber, and the size difference between these two is small. In addition, the first panel and/or the second panel are provided with a notch which is located at the opening of the sidewall. The notch can facilitate the installation of atomizing sheet. According to an embodiment of the present invention, the sealing sleeve has a diameter of 10-50 mm and a thickness of 1-10 mm, and the angle of the opening on the sidewall is 30-150°.

According to an embodiment of the present invention, a plurality of reinforcing bars are arranged along the radial direction on the outer surface of the first panel or the outer surface of the second panel. According to another embodiment of the present invention, a plurality of reinforcing bars are arranged along the radial direction on the outer surface of the first panel and the outer surface of the second panel. The so-called radial direction represents the direction from the center to the periphery. There is no special restriction on the number and the shape of reinforcing bars. In this way, the strength of the sealing sleeve can be improved without affecting the installation of the atomizing sheet. The surfaces of the first panel and the second panel that are used to form the atomizing sheet chamber are the inner surfaces, and the surfaces opposite to the inner surfaces are the outer surfaces. According to an embodiment of the present invention, a plurality of through holes can be arranged on the side wall as long as the sealing of the whole medical atomizer is not affected.

In the present invention, the atomizing sheet is located between the first panel and the second panel, and does not exceed the area defined by the sidewall. In this way, the core component, i.e. the atomizing sheet, can be protected from external interference.

In the present invention, a first opening is arranged in the middle of the first panel, and a second opening is arranged in the middle of the second panel; the first opening and the second opening are used cooperatively so as to make at least a part of the atomizing sheet expose outside the atomizing sheet chamber. That is to say, the first panel and the second panel cannot completely block the atomizing sheet, especially the microporous film of the atomizing sheet. For example, the first opening and the second opening are arranged oppositely, so as to ensure that the microporous film of the atomizing sheet is exposed outside the atomizing sheet chamber. The shape of the first opening and the second opening is preferred to be circular. The diameters of the first opening and the second opening can be 3-15 mm, preferably 3-12 mm. In some embodiment, the diameter of the first opening (D11) is different from the diameter of the second opening (D12). For example, the diameter of the first opening (D11) is larger than that of the second opening (D12). D11-D12 can be 1-6 mm, preferably 2-5 mm. This is helpful to the vibration of the microporous film of the atomizing sheet, thus the atomization effect is improved.

In the present invention, the inner side of the first opening is provided with a first sealing ring, and the inner side of the second opening is provided with a second sealing ring;
the first sealing ring and the second sealing ring are matched for fixing the atomizing sheet in the atomizing sheet chamber. By providing a sealing ring on the first panel or the second panel, the atomizing sheet can be tightly combined with the sealing sleeve, and the sealing sleeve can be tightly combined with the drug storage device and the atomized drug collecting tank, thus the sealing performance of the medical atomizer is improved. The first sealing ring and the second sealing ring may be O-sealing rings. The diameters of the first sealing ring and the second sealing ring can be 2-10 mm, preferably 5-9 mm. The wire diameters of the first sealing ring and the second sealing ring can be 0.5-4 mm, preferably 0.5-2 mm. This is helpful to the vibration of the microporous film of the atomizing sheet, and ensures the sealing effect. In some embodiment, the diameter of the first seal ring (D111) is different from the diameter of the second seal ring (D121). For example, the diameter of the first seal ring (Dill) is larger than that of the second seal ring (D121). D111-D121 can be 0.5-6 mm, preferably 1-5 mm. This is helpful to the vibration of the microporous film of the atomizing sheet, and ensures the sealing effect. The wire diameters of the first sealing ring and the second sealing ring may be the same or different, preferably the same wire diameter represents the maximum thickness of the sealing ring body.

In the present invention, the sidewall, the first panel, the second panel, the first sealing ring and the second sealing ring form a solid whole. According to an embodiment of the present invention, the sidewall, the first panel, the second panel, the first sealing ring and the second sealing ring are integral structure. They can be made of the same or different materials. Preferably, the material of the sidewall, the first panel, the second panel, the first seal ring and the second seal ring are the same. These materials include, but are not limited to, polymer materials such as silicon rubber and the like.

### <Auxiliary Components>

The medical atomizer in the present invention may comprise a three-way pipe, a one-way valve and a mouthpiece. The three-way pipe comprises a first end, a second end and a third end. The first end is connected with the lower part of the lower atomized drug collecting section for the extraction of atomized drug. The second end is open to the atmosphere to avoid the air flow being too small to make users comfort. The third end is communicated with the mouthpiece for users to grip. There is no special restriction on the material of the three-way pipe and the mouthpiece, as long as the material is one of medical materials. The material of the three-way pipe and the mouthpiece can be the same or different. The one-way valve is located in the three-way pipe, which is arranged to be able to lead out the atomized drug to the mouthpiece and prevent air from entering the atomized drug collecting tank. Those known in the field can be used as a one-way valve, which will not be described in details herein.

The medical atomizer in the present invention may also comprise a power supply, a wire and a plug. The power supply is connected with the plug through the wire. Examples of power supply include, but are not limited to, rechargeable lithium batteries, alkaline batteries, and DC power supplies. The plugging component is arranged to be able to accommodate the plug, and be able to electrically connect the atomizing sheet with the plug. For example, the plug is electrically connected to the conductive spring on the atomizing sheet, so that power is supplied to the atomizing sheet. The shape of the plug matches the shape of the plugging component. There are no special restrictions on the shape of the plug, such as rectangular or nearly rectangular. The present invention designs the atomization part and the power supply separately, so that it is convenient to be used.

### Example 1

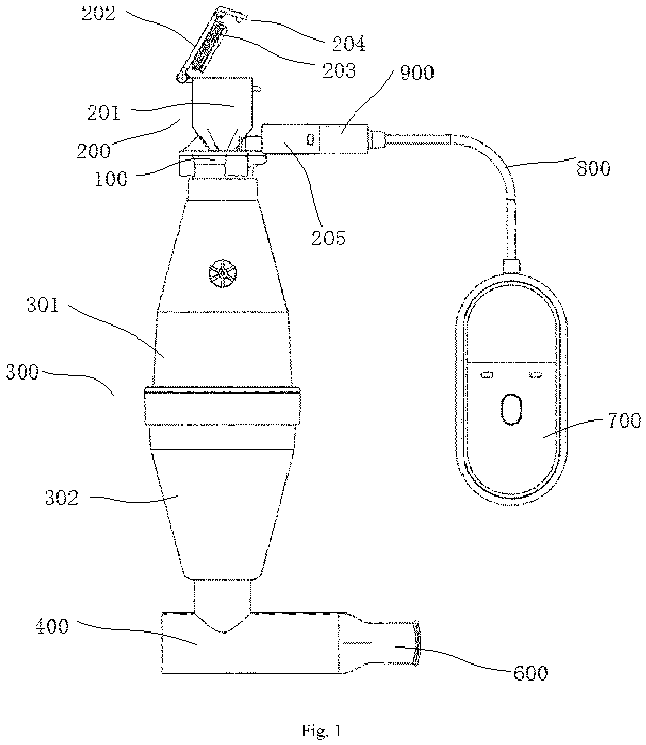

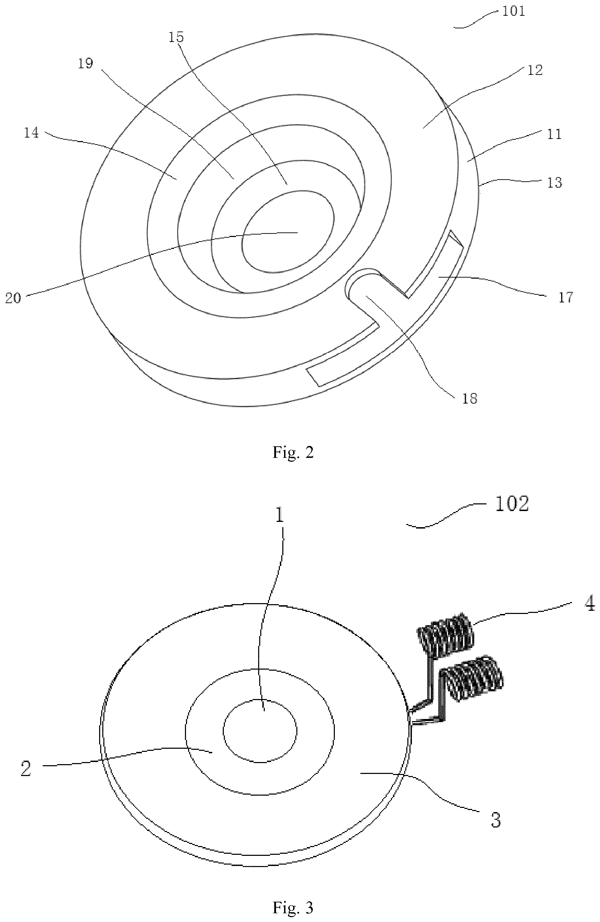

Fig. 1 is a schematic diagram of a medical atomizer of the present invention. The medical atomizer comprises an atomization generating assembly 100, a drug storage device 200 and an atomized drug collecting tank 300. The lower part of the drug storage device 200 is provided with a first socket structure (socket). The atomized drug collecting tank 300 has a shape of spindle, and comprises an upper atomized drug collecting section 301 and a lower atomized drug collecting section 302 which are connected with each other through threads. The upper part of the upper atomized drug collecting section 301 is provided with a second socket structure (socket tube). The heights of the first socket structure and the second socket structure are both 5 mm. The first socket structure forms a socket connection with the second socket structure. The first socket structure and the second socket structure cooperate with each other to form an accommodation chamber for fixing the atomization generating assembly 100. As shown in Fig. 2, the first sealing ring 14 on the first panel 12 and the second sealing ring 15 on the second panel 13 are tightly combined with the accommodation chamber, so that the sealing sleeve 101 is tightly combined with the drug storage device 200 and the atomized drug collecting tank 300, thus the sealing performance of medical atomizer is improved.

As shown in Fig. 1, the upper part of the drug storage device 200 comprises an atomizing cup body 201, an atomizing cup cover 202, a sealing member 203, a clasp 204 and a plugging component 205. The atomizing cup cover 202 is arranged above the atomizing cup body 201 and is connected with the atomizing cup body 201. The sealing member 203 is a silicon rubber sealing ring, which is arranged on the lower surface of the atomizing cup cover 202, and is located between the atomizing cup body 201 and the atomizing cup cover 202, so as to seal these two. The clasp 204 is arranged on the side of the atomizing cup cover 202, so that the atomizing cup cover 202 can be firmly fastened on the atomizing cup body 201. The plugging component 205 is arranged on one side of the atomizing cup body 201, and is connected with the accommodation chamber.

The medical atomizer also comprises a three-way pipe 400, a one-way valve (not shown in the figure) and a mouthpiece 600. The three-way pipe 400 comprises a first end, a second end and a third end, the first end is connected with the lower part of the lower atomized drug collecting section 302, the second end is open to the atmosphere, and the third end is communicated with the mouthpiece 600. The one-way valve is located in the three-way pipe 400, which is arranged to be able to lead out the atomized drug from the atomized drug collecting tank 300 to the mouthpiece 600 and prevent air from entering the atomized drug collecting tank 300.

As shown in Fig. 1, the medical atomizer also comprises a power supply 700, a wire 800 and a plug 900. The power supply 700 is connected with the plug 900 through the wire 800. The plugging component 205 is arranged to be able to accommodate the plug 900, and be able to electrically connect the atomizing sheet 102 with the plug 900.

Fig. 2 and Fig. 3 are schematic diagrams of a sealing sleeve and an atomizing sheet of an atomization generating assembly of the present invention, respectively. The atomization generating assembly 100 comprises a sealing sleeve 101 and an atomizing sheet 102. The atomizing sheet 102 can be accommodated in the sealing sleeve 101. As shown in Fig. 3, the atomizing sheet 102 comprises microporous membrane 1, conductive sheet 2, silicon rubber sheath 3 and two conductive springs 4. The conductive sheet 2 is arranged in the silicon rubber sheath 3, the microporous membrane 1 is arranged in the central hole of the conductive sheet 2, and is electrically connected with the conductive sheet 2. The conductive spring 4 is electrically connected with the conductive sheet 2. The microporous membrane 1 is in contact with the liquid medicine and has many micropores for atomizing the liquid medicine into a mist.

As shown in Fig. 2, the sealing sleeve 101 comprises a sidewall 11, a first panel 12, a second panel 13, a first sealing ring 14 and a second sealing ring 15. They are integral structure and made of silicon rubber. The first panel 11 and the second panel 12 are arranged oppositely; the sidewall 11, the first panel 12 and the second panel 13 are jointly enclosed to form an atomizing sheet chamber for installing the atomizing sheet 102. The atomizing sheet 102 is located between the first panel 12 and the second panel 13, and it does not exceed the area defined by the sidewall 11. The first panel 12 and the second panel 13 are annular structures, while the sidewall 13 is circumferential structure. The sealing sleeve has a diameter of 19 mm and a thickness of 3 mm. The atomizing sheet chamber has a diameter of 18 mm. The atomizing sheet has a diameter of 16 mm

As shown in Figure 2, an opening 17 is arranged on the sidewall 11. The angle of the opening is 90°, so that the atomizing sheet 102 can be installed in the atomizing sheet chamber easily. A notch 18 is arranged on the first panel 12, which is located at the opening 17. A circular first opening 19 is arranged in the middle of the first panel 12, and a circular second opening 20 is arranged in the middle of the second panel 13; the diameter of the former is larger than that of the latter. They are used cooperatively so as to ensure the microporous membrane 1 of the atomizing sheet 102 exposed outside the atomizing sheet chamber. The first sealing ring 14 is arranged on the inner side of the first opening 19, and the second sealing ring 15 is arranged on the inner side of the second opening 20. They are used cooperatively so as to ensure that the atomizing sheet 102 is fixed in the atomizing sheet chamber. The first sealing ring 14 and the second sealing ring 15 are sealing rings with a shape of O-ring. The first sealing ring 14 has a diameter of 9 mm and a wire diameter of 1 mm. The second sealing ring 15 has a diameter of 6 mm and a wire diameter of 1 mm.

### Example 2

The components and connection relations are the same as those in Example 1, except that the first socket structure was changed into a socket tube and the second socket structure was changed into a socket.

The present invention is not limited by the above embodiments. All variations, modifications and replacements to the disclosed embodiments which are apparent to those skilled in the art and do not depart from the essence of the present invention fall in the scope of the present invention.

## Claims

1. A medical atomizer for atomization inhalation therapy, comprising a drug storage device (200), an atomized drug collecting tank (300) and an atomization generating assembly (100) , an accommodation chamber is formed between the lower part of the drug storage device (200) and the atomized drug collecting tank (300), the accommodation chamber is used for accommodating the atomization generating assembly (100);
wherein the atomization generating assembly (100) comprises a sealing sleeve (101) and an atomizing sheet (102); the sealing sleeve (101) has a sidewall, a first panel (12) and a second panel (13) , the first panel (12) and the second panel (13) are arranged oppositely, the sidewall is arranged to connect the first panel (12) and the second panel (13) into an integral structure, the sidewall, the first panel (12) and the second panel (13) are jointly enclosed, forming an atomizing sheet (102) chamber which is used to accommodate the atomizing sheet (102);
the atomizing sheet (102) is located between the first panel (12) and the second panel (13), and it does not exceed the area defined by the sidewall; the middle part of the first panel (12) is provided with a first opening (19), the middle part of the second panel (13) is provided with a second opening (20); the first opening (19) and the second opening (20) are used cooperatively so as to make at least a part of the atomizing sheet (102) exposed outside the atomizing sheet (102) chamber;
wherein a first sealing ring (14) is arranged on the inner side of the first opening (19), and a second sealing ring (15) is arranged on the inner side of the second opening (20); the first sealing ring (14) and the second sealing ring (15) are matched for fixing the atomizing sheet (102) in the atomizing sheet (102) chamber;
wherein the first panel (12), the second panel (13), the first sealing ring (14) and the second sealing ring (15) are formed into integral structure;
**characterized in that**
the sidewall is provided with an opening (17) for installing the atomizing sheet (102) into the atomizing sheet chamber, the first panel (12) and/or the second
panel (13) are provided with a notch (18) which is located at the opening (17) on the sidewall,
and the shape of the atomized drug collection tank (300) is spindle.

2. The medical atomizer according to claim 1, **characterized in that** the lower part of the drug storage device (200) is provided with a first socket structure, the atomized drug collecting tank (300) comprises an upper atomized drug collecting section (301) and a lower atomized drug collecting section (302), the upper part of the upper atomized drug collecting section (301) is provided with a second socket structure, the first socket structure forms a socket connection with the second socket structure; both the first socket structure and the second socket structure have a height of 1-20 mm.

3. The medical atomizer according to claim 2, **characterized in that** the medical atomizer also comprises a three-way pipe (400), a one-way valve and a mouthpiece (600); the three-way pipe (400) comprises a first end, a second end and a third end, the first end is connected with the lower part of the lower atomized drug collecting section (302), the second end is open to the atmosphere, and the third end is communicated with the mouthpiece (600); the one-way valve is located in the three-way pipe (400), which is arranged to be able to lead out the atomized drug to the mouthpiece (600) and prevent air from entering the atomized drug collecting tank (300).

4. The medical atomizer according to claim 3, **characterized in that** the upper part of the drug storage device (200) comprises an atomizing cup body, an atomizing cup cover, a sealing member (203), a clasp (204) and a plugging component (205); the atomizing cup cover is arranged above the atomizing cup body and is connected with the atomizing cup body; the sealing member (203) is arranged on the lower surface of the atomizing cup cover for sealing the atomizing cup body; the clasp (204) is arranged on the side of the atomizing cup cover or the atomizing cup body; the plugging component (205) is arranged on one side of the atomizing cup body, and is connected with the accommodation chamber.

5. The medical atomizer according to claim 4, **characterized in that** the medical atomizer also comprises a power supply (700), a wire (800) and a plug (900); the power supply (700) is connected with the plug (900) through the wire (800); and the plugging component (205) is arranged to be able to accommodate the plug (900), and be able to electrically connect the atomizing sheet (102) with the plug (900).

6. The medical atomizer according to any one of claims 1-5, **characterized in that** both the first panel (12) and the second panel (13) are in annular shape, the sidewall (11) is a circumferential sidewall (11); and the diameter of the first opening (19) is different from that of the second opening (20).

7. The medical atomizer according to claim 6, **characterized in that** the sealing sleeve (101) has a diameter of 10-50 mm and a thickness of 1-10 mm, and the angle of the opening (17) on the sidewall (11) is 30-150 degrees.

8. The medical atomizer according to claim 7, **characterized in that** both the first sealing ring (14) and the second sealing ring (15) are O-sealing rings with diameters of 2-10 mm and wire (800) diameters of 0.5-4 mm.

## Patentansprüche

1. Medizinischer Zerstäuber für die Zerstäubungsinhalationstherapie, umfassend eine Medikamentenspeichervorrichtung (200), einen Sammelbehälter (300) für zerstäubte Medikamente und eine Zerstäubung-erzeugende Anordnung (100), wobei eine Aufnahmekammer zwischen dem unteren Teil der Medikamentenspeichervorrichtung (200) und dem Sammelbehälter (300) für zerstäubte Medikamente ausgebildet ist, wobei die Aufnahmekammer zur Aufnahme der die Zerstäubung erzeugenden Anordnung (100) vorgesehen ist;
wobei die Zerstäubung-erzeugende Anordnung (100) eine Dichtungshülse (101) und ein Zerstäubungsblatt (102) umfasst; wobei die Dichtungshülse (101) eine Seitenwand, ein erstes Paneel (12) und ein zweites Paneel (13) aufweist, wobei das erste Paneel (12) und das zweite Paneel (13) gegenüberliegend angeordnet sind, wobei die Seitenwand so angeordnet ist, dass sie das erste Paneel (12) und das zweite Paneel (13) zu einer integralen Struktur verbindet, wobei die Seitenwand, das erste Paneel (12) und das zweite Paneel (13) gemeinsam eingeschlossen sind und eine Kammer des Zerstäubungsblatts (102) ausbilden, die zur Aufnahme des Zerstäubungsblatts (102) vorgesehen ist; wobei das Zerstäubungsblatt (102) zwischen dem ersten Paneel (12) und dem zweiten Paneel (13) angeordnet ist und den durch die Seitenwand eingeschränkten Bereich nicht überschreitet; wobei der mittlere Teil des ersten Paneels (12) mit einer ersten Öffnung (19) versehen ist, der mittlere Teil des zweiten Paneels (13) mit einer zweiten Öffnung (20) versehen ist; wobei die erste Öffnung (19) und die zweite Öffnung (20) zusammenwirkend eingestellt werden, sodass zumindest ein Teil des Zerstäubungsblatts (102) außerhalb der Kammer des Zerstäubungsblatts (102) freilegt;
wobei ein erster Dichtungsring (14) an der Innenseite der ersten Öffnung (19) angeordnet ist und ein zweiter Dichtungsring (15) an der Innenseite der zweiten Öffnung (20) angeordnet ist; wobei der erste Dichtungsring (14) und der zweite Dichtungsring (15) zum Fixieren des Zerstäubungsblatts (102) in der Kammer des Zerstäubungsblatts (102) angepasst sind;
wobei das erste Paneel (12), das zweite Paneel (13), der erste Dichtungsring (14) und der zweite Dichtungsring (15) als integrale Struktur ausgebildet sind;
**dadurch gekennzeichnet, dass** die Seitenwand mit einer Öffnung (17) zum Einbringen des Zerstäubungsblatts (102) in die Kammer des Zerstäubungsblatts versehen ist,
wobei das erste Paneel (12) und/oder das zweite Paneel (13) mit einer Kerbe (18) versehen sind, die sich an der Öffnung (17) an der Seitenwand befindet, und wobei die Form des Sammelbehälters (300) für zerstäubte Medikamente spindelförmig ist.

2. Medizinischer Zerstäuber nach Anspruch 1, **dadurch gekennzeichnet, dass** der untere Teil der Medikamentenspeichervorrichtung (200) mit einer ersten Sockelstruktur versehen ist, wobei der Sammelbehälter (300) für zerstäubten Medikamente einen oberen Abschnitt (301) zum Sammeln der zerstäubten Medikamente und einen unteren Abschnitt (302) zum Sammeln der zerstäubten Medikamente umfasst, wobei der obere Teil des oberen Abschnitts (301) zum Sammeln der zerstäubten Medikamente mit einer zweiten Sockelstruktur versehen ist, wobei die erste Sockelstruktur eine Sockelverbindung mit der zweiten Sockelstruktur bildet; sowohl die erste Sockelstruktur als auch die zweite Sockelstruktur eine Höhe von 1-20 mm aufweisen.

3. Medizinischer Zerstäuber nach Anspruch 2, **dadurch gekennzeichnet, dass** der medizinische Zerstäuber auch ein Drei-Wege-Rohr (400), ein Einwegventil und ein Mundstück (600) umfasst; wobei das Drei-Wege-Rohr (400) ein erstes Ende, ein zweites Ende und ein drittes Ende umfasst, wobei das erste Ende mit dem unteren Teil des unteren Abschnitt (302) zum Sammeln der zerstäubten verbunden ist, das zweite Ende zur Atmosphäre hin offen ist und das dritte Ende mit dem Mundstück (600) in Verbindung steht; wobei das Einwegventil sich in dem Drei-Wege-Rohr (400) befindet, das so angeordnet ist, dass sie das zerstäubte Medikament zum Mundstück (600) herauszuführen und das Eintritten der Luft in den Sammelbehälter (300) für das zerstäubte Medikament verhindern kann.

4. Medizinischer Zerstäuber nach Anspruch 3, **dadurch gekennzeichnet, dass** der obere Teil der Medikamentenspeichervorrichtung (200) einen Zerstäubungsbecherkörper, einen Zerstäubungsbecherdeckel, ein Dichtungselement (203), eine Spange (204) und eine Steckkomponente (205) umfasst; wobei der Zerstäubungsbecherdeckel oberhalb des Zerstäubungsbecherkörpers angeordnet und mit dem Zerstäubungsbecherkörper verbunden ist; wobei das Dichtungselement (203) an der Unterseite des Zerstäubungsbecherdeckels zum Abdichten des Zerstäubungsbecherkörpers angeordnet ist; wobei die Spange (204) an der Seite des Zerstäubungsbecherdeckels oder des Zerstäubungsbecherkörpers angeordnet ist; wobei die Steckkomponente (205) an einer Seite des Zerstäubungsbecherkörpers angeordnet und mit der Aufnahmekammer verbunden ist.

5. Medizinischer Zerstäuber nach Anspruch 4, **dadurch gekennzeichnet, dass** der medizinische Zerstäuber auch eine Stromversorgung (700), einen Draht (800) und einen Stecker (900) umfasst; wobei die Stromversorgung (700) mit dem Stecker (900) durch den Draht (800) verbunden ist; und die Steckkomponente (205) so angeordnet ist, dass sie den Stecker (900) aufnehmen kann, und sie das Zerstäubungsblatt (102) mit dem Stecker (900) elektrisch verbinden kann.

6. Medizinischer Zerstäuber nach einem der Ansprüche 1 bis 5, **dadurch gekennzeichnet, dass** sowohl das erste Paneel (12) als auch das zweite Paneel (13) ringförmig sind, wobei die Seitenwand (11) eine Umfangsseitenwand (11) ist; und der Durchmesser der ersten Öffnung (19) sich von dem der zweiten Öffnung (20) unterscheidet.

7. Medizinischer Zerstäuber nach Anspruch 6, **dadurch gekennzeichnet, dass** die Dichtungshülse (101) einen Durchmesser von 10-50 mm und eine Dicke von 1-10 mm aufweist, und wobei der Winkel der Öffnung (17) an der Seitenwand (11) 30-150 Grad beträgt.

8. Medizinischer Zerstäuber nach Anspruch 7, **dadurch gekennzeichnet, dass** sowohl der erste Dichtungsring (14) als auch der zweite Dichtungsring (15) O-Dichtungsringe mit Durchmessern von 2-10 mm und Drahtdurchmessern (800) von 0,5-4 mm sind.

## Revendications

1. Un atomiseur médical pour inhalothérapie par atomisation, comprenant un dispositif de stockage de médicament (200), un réservoir de collecte de médicament atomisé (300) et un ensemble de génération d'atomisation (100), une chambre de logement est formée entre la partie inférieure du dispositif de stockage de médicament (200) et le réservoir de collecte de médicament atomisé (300), la chambre de logement est utilisée pour loger l'ensemble de génération d'atomisation (100);
dans lequel l'ensemble de génération d'atomisation (100) comprend un manchon d'étanchéité (101) et une feuille d'atomisation (102); le manchon d'étanchéité (101) a une paroi latérale, un premier panneau (12) et un deuxième panneau (13), le premier panneau (12) et le deuxième panneau (13) sont disposés de manière opposée, la paroi latérale est disposée pour relier le premier panneau (12) et le deuxième panneau (13) en une structure intégrale, la paroi latérale, le premier panneau (12) et le deuxième panneau (13) sont enfermés conjointement, formant une chambre de feuille d'atomisation (102) qui est utilisée pour loger la feuille d'atomisation (102); la feuille d'atomisation (102) se trouve entre le premier panneau (12) et le second panneau (13), et elle ne dépasse pas la zone définie par la paroi latérale; la partie médiane du premier panneau (12) est munie d'une première ouverture (19), la partie médiane du deuxième panneau (13) est munie d'une deuxième ouverture (20); la première ouverture (19) et la seconde ouverture (20) sont utilisées en coopération de manière à rendre au moins une partie de la feuille d'atomisation (102) exposée à l'extérieur de la chambre de la feuille d'atomisation (102);
dans lequel un premier anneau d'étanchéité (14) est disposé sur le côté intérieur de la première ouverture (19), et un deuxième anneau d'étanchéité (15) est disposé sur le côté intérieur de la deuxième ouverture (20); la première anneau d'étanchéité (14) et la seconde anneau d'étanchéité (15) sont appariées pour fixer la feuille d'atomisation (102) dans la chambre de feuille d'atomisation (102);
dans lequel le premier panneau (12), le deuxième panneau (13), la première anneau d'étanchéité (14) et la deuxième anneau d'étanchéité (15) sont formées en une structure intégrale;
**caractérisé en ce que**
la paroi latérale est pourvue d'une ouverture (17) pour installer la feuille d'atomisation (102) dans la chambre de feuille d'atomisation,
le premier panneau (12) et/ou le deuxième panneau (13) sont pourvus d'une encoche (18) qui se trouve au niveau de l'ouverture (17) sur la paroi latérale, et la forme du réservoir de collecte de médicament atomisé (300) est fusiforme.

2. L'atomiseur médical selon la revendication 1, **caractérisé en ce que** la partie inférieure du dispositif de stockage de médicament (200) est pourvue d'une première structure de douille, le réservoir de collecte de médicament atomisé (300) comprend une section supérieure de collecte de médicament atomisé (301) et une section inférieure de collecte de médicament atomisé (302), la partie supérieure de la section supérieure de collecte de médicament atomisé (301) est pourvue d'une seconde structure de douille, la première structure de douille forme une connexion de douille avec la seconde structure de douille; à la fois la première structure de douille et la seconde structure de douille ont une hauteur de 1-20 mm.

3. L'atomiseur médical selon la revendication 2, **caractérisé en ce que** l'atomiseur médical comprend également un tuyau à trois voies (400), une soupape unidirectionnelle et un embout buccal (600); le tuyau à trois voies (400) comprend une première extrémité, une deuxième extrémité et une troisième extrémité, la première extrémité est reliée à la partie inférieure de la section inférieure de collecte de médicament atomisé (302), la deuxième extrémité est ouverte à l'atmosphère, et la troisième extrémité est en communication avec l'embout buccal (600); la soupape unidirectionnelle se trouve dans le tuyau à trois voies (400), qui est disposé pour pouvoir faire sortir le médicament atomisé vers l'embout buccal (600) et empêcher l'air d'entrer dans le réservoir collecteur de médicament atomisé (300).

4. L'atomiseur médical selon la revendication 3, **caractérisé en ce que** la partie supérieure du dispositif de stockage de médicament (200) comprend un corps de coupelle d'atomisation, un couvercle de coupelle d'atomisation, un élément d'étanchéité (203), un fermoir (204) et un élément d'obturation (205); le couvercle de coupelle d'atomisation est disposé au-dessus du corps de coupelle d'atomisation et est relié au corps de coupelle d'atomisation; l'élément d'étanchéité (203) est disposé sur la surface inférieure du couvercle de coupelle d'atomisation pour étanchéifier le corps de coupelle d'atomisation; le fermoir (204) est disposé sur le côté du couvercle de coupelle d'atomisation ou le corps de coupelle d'atomisation; le élément d'obturation (205) est disposé sur un côté du corps de coupelle d'atomisation et est relié à la chambre de logement.

5. L'atomiseur médical selon la revendication 4, **caractérisé en ce que** l'atomiseur médical comprend également une alimentation électrique (700), un fil (800) et un bouchon (900); l'alimentation électrique (700) est connectée au bouchon (900) à travers le fil (800); et l'élément d'obturation (205) est disposé pour pouvoir loger le bouchon (900), et être capable de connecter électriquement la feuille d'atomisation (102) au bouchon (900).

6. L'atomiseur médical selon l'une quelconque des revendications 1 à 5, **caractérisé en ce qu'**à la fois le premier panneau (12) et le second panneau (13) sont de forme annulaire, la paroi latérale (11) est une paroi latérale (11) circonférentielle; et le diamètre de la première ouverture (19) est différent de celui de la deuxième ouverture (20).

7. L'atomiseur médical selon la revendication 6, **caractérisé en ce que** le manchon d'étanchéité (101) a un diamètre de 10-50 mm et une épaisseur de 1-10 mm, et l'angle de l'ouverture (17) sur la paroi latérale (11) est 30-150 degrés.

8. L'atomiseur médical selon la revendication 7, **caractérisé en ce que** la première anneau d'étanchéité (14) et la seconde anneau d'étanchéité (15) sont des anneau d'étanchéité en form de O avec des diamètres de 2-10 mm et des diamètres de fil (800) de 0,5-4 mm
